(19) Europäisches Patentamt
European Patent Office
Office européen des brevets

(11) Numéro de publication : **0 447 292 A1**

# (12) DEMANDE DE BREVET EUROPEEN

(21) Numéro de dépôt : **91400585.5**

(51) Int. Cl.[5] : **C07D 211/26**, A61K 31/445

(22) Date de dépôt : **04.03.91**

(30) Priorité : **07.03.90 FR 9002855**

(43) Date de publication de la demande :
**18.09.91 Bulletin 91/38**

(84) Etats contractants désignés :
**AT BE CH DE DK ES FR GB GR IT LI LU NL SE**

(71) Demandeur : **SYNTHELABO**
**58 rue de la Glacière**
**F-75013 Paris (FR)**

(72) Inventeur : **George, Pascal**
**19, rue des Quatre Vents**
**F-78730 St Arnoult en Yvelines (FR)**
Inventeur : **Froissant, Jacques**
**Cidex 981**
**Brevainville, F-41160 Moree (FR)**
Inventeur : **Sevrin, Mireille**
**73, rue Raymond Losserand**
**F-75014 Paris (FR)**

(74) Mandataire : **Ludwig, Jacques et al**
**SYNTHELABO, Service Brevets, 22, avenue Galilée, B.P. 72**
**F-92352 Le Plessis Robinson Cédex (FR)**

(54) **Dérivés de 4-(aminométhyl)pipéridine, leur préparation et leur application en thérapeutique.**

(57) Composés de formule générale (I)

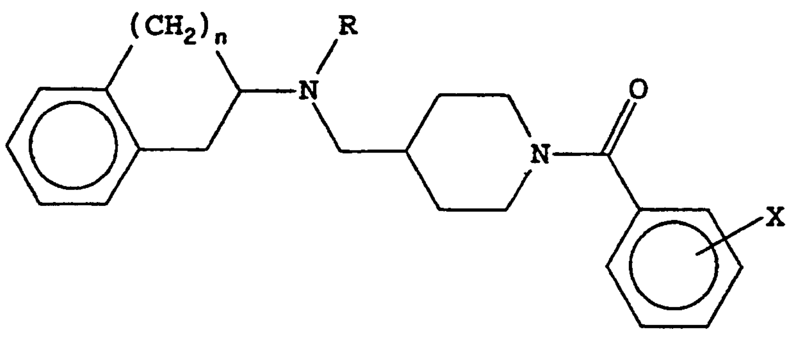

(I)

dans laquelle
n représente le nombre 1 ou 2, et
R représente un groupe alkyle linéaire ou ramifié en $C_1$-$C_3$ et
X représente un ou plusieurs substituants choisis parmi les atomes d'hydrogène et d'halogène et les groupes alkyles en $C_1$-$C_3$ et alcoxy en $C_1$-$C_3$.
Application en thérapeutique.

EP 0 447 292 A1

# DERIVES DE 4-(AMINOMETHYL)PIPERIDINE, LEUR PREPARATION ET LEUR APPLICATION EN THERAPEUTIQUE

La présente invention a pour objet des dérivés de 4-(aminométhyl)pipéridine, leur préparation et leur application en thérapeutique.

Les composés de l'invention répondent à la formule générale (I) du schéma donné ci-après, formule dans laquelle
n représente le nombre 1 ou 2, et
R représente un groupe alkyle linéaire ou ramifié en $C_1$-$C_3$, et
X représente un ou plusieurs substituants choisis parmi les atomes d'hydrogène et d'halogène et les groupes alkyles en $C_1$-$C_3$ et alcoxy en $C_1$-$C_3$.

Ils peuvent se présenter à l'état de bases libres ou de sels d'addition à des acides.

Un procédé de préparation des composés de l'invention est illustré par le schéma donné ci-après.

On fait réagir d'abord une amine primaire de formule générale (II) (dans laquelle n est tel que défini ci-dessus), éventuellement sous forme de sel d'addition avec un acide, soit (a) avec un chloroformiate d'alkyle ou de benzyle, dans un solvant aprotique inerte tel que le toluène, le tétrahydrofurane, ou un solvant halogéné, par exemple le dichlorométhane, et en présence d'une base organique telle qu'une amine tertiaire, par exemple la triéthylamine, soit (b) avec un chlorure d'acide en $C_2$ ou $C_3$, dans le même type de solvants et en présence du même type de bases.

Dans le premier cas (a) on obtient un carbamate d'alkyle ou de benzyle qui, par réduction, par exemple avec l'hydrure de lithium et d'aluminium, dans un solvant éthéré tel que le tétrahydrofurane, fournit une amine secondaire de formule générale (III) dans laquelle R représente un groupe méthyle. Dans le second cas (b) on obtient un amide qui, par réduction, par exemple avec l'hydrure de lithium et d'aluminium, dans un solvant éthéré tel que le tétrahydrofurane, fournit une amine secondaire de formule générale (III) dans laquelle R représente un groupe alkyle en $C_2$ ou $C_3$.

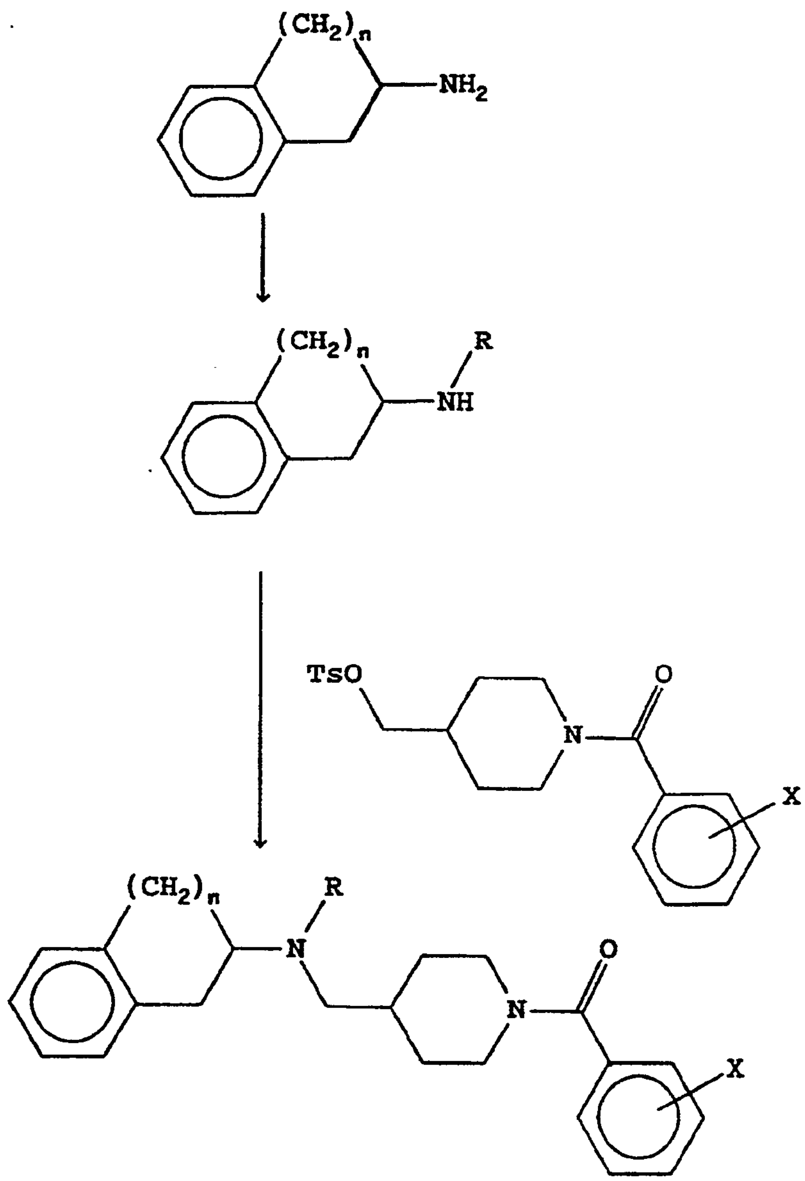

(II)

(III)

(IV)

(I)

On fait ensuite réagir l'amine secondaire avec un tosylate de formule (IV) (dans laquelle Ts représente un groupe (4-méthylphényl)sulfonyle), soit en l'absence, soit en présence d'un solvant inerte tel que le diméthylformamide, le toluène ou le xylène, à une température de 20 à 150°C, et éventuellement en présence d'une base organique, telle qu'une amine tertiaire, ou minérale, telle qu'un carbonate ou hydrogéno-carbonate alcalin.

L'amine de départ de formule générale (II) dans laquelle n=1 est disponible dans le commerce (indan-2-amine) ; l'amine de départ de formule générale (II) dans laquelle n=2 peut être préparée selon des méthodes connues, à partir de 3,4-dihydro-(1*H*)naphtalén-2-one, disponible dans le commerce, par réaction avec le chlorhydrate d'hydroxylamine en présence d'acétate de sodium, puis hydrogénation catalytique de l'oxime intermédiaire.

Le tosylate de formule (IV) peut être préparé par exemple comme décrit dans la demande de brevet EP-0306375.

Les exemples suivants illustrent en détail la préparation de quelques composés selon l'invention. Les microanalyses élémentaires et les spectres IR et RMN confirment les structures des produits obtenus.

Exemple 1

*N*-(indan-2-yl)-*N*-méthyl-1-benzoylpipéridine-4-méthanamine, chlorhydrate.

a) Indane-2-carbamate d'éthyle.

A 5 g (29,5 mmoles) de chlorhydrate d'indan-2-amine on ajoute 200 ml de dichlorométhane puis 10 ml, soit 7,26 g (71,7 mmoles) de triéthylamine. On agite le mélange pendant 30mn, puis on ajoute, goutte à goutte, 3,4 ml, soit 3,84 g (35,4 mmoles) de chloroformiate d'éthyle. On agite le mélange pendant 30mn, puis on ajoute de l'eau glacée et un excès d'acide chlorhydrique 1N. On filtre le précipité, on le lave, on le sèche et on le purifie par chromatographie sur colonne de gel de silice en éluant avec un mélange 80/20 de dichlorométhane/acétate d'éthyle. On isole 4,25 g de solide amorphe. Point de fusion : 68-68,5°C.

b) *N*-méthylindan-2-amine.

A une suspension de 1,13 g (30 mmoles) d'hydrure de lithium et d'aluminium anhydre dans 150 ml de tétrahydrofurane on ajoute une solution de 2,2 g d'indane-2-carbamate d'éthyle dans 50 ml du même solvant. On chauffe le mélange au reflux, on le laisse refroidir, on l'hydrolyse avec un excès de soude aqueuse 1N, on filtre le mélange, on le concentre et on le distille sous environ 100 Pa à 150°C. On obtient 3,5 g d'huile.

c) *N*-(indan-2-yl)-*N*-méthyl-1-benzoylpipéridine-4-méthanamine, chlorhydrate.

Dans un ballon de 50 ml placé sous azote on chauffe, au bain d'huile à 150°C, un mélange de 1,13 g (7,7 mmoles) de *N*-méthylindan-2-amine et 2,9 g (7,7 mmoles) de 4-méthylphényl-sulfonate de (1-benzoylpipéridin-4-yl)méthyle pendant 3h. On laisse le mélange revenir à température ambiante, on ajoute du dichlorométhane et un excès de soude aqueuse 1N. Après dissolution complète du milieu réactionnel, on sépare la phase organique, on évapore le solvant et on purifie le résidu par chromatographie sur colonne de gel de silice en éluant avec un mélange 97/3 de dichlorométhane/méthanol. On obtient 1,3 g d'amide auquel on ajoute 3 ml d'acide chlorhydrique 1,7M dans l'éthanol, on agite le mélange pendant 15mn, on l'évapore à sec, et on recristallise le solide dans l'acétonitrile puis dans l'acétone. On isole finalement 0,95 g de chlorhydrate.
Point de fusion : 212-213°C.

Exemple 2

*N*-(indan-2-yl)-*N*-propyl-1-benzoylpipéridine-4-méthanamine, chlorhydrate.

a) *N*-(indan-2-yl)propionamide.

A 5 g (29,5 mmoles) de chlorhydrate d'indan-2-amine on ajoute 200 ml de dichlorométhane puis 15 ml, soit 10,9 g (107,6 mmoles) de triéthylamine. On agite le mélange puis on ajoute 2,6 ml (29,5 mmoles) de chlorure de propionyle et on maintient l'agitation pendant 12h à température ambiante. On ajoute de l'acide chlorhydrique dilué, on filtre le précipité, on le lave, on le sèche et on le purifie par chromatographie sur colonne de gel de silice en éluant avec un mélange 80/20 de dichlorométhane/acétate d'éthyle. On isole 4,87 g de solide beige.

b) *N*-propylindan-2-amine.

Dans les conditions décrites à propos de la *N*-méthylindan-2-amine, on réduit 4,87 g (25,7 mmoles) de *N*-(indan-2-yl)propionamide dans le tétrahydrofurane au moyen de 1,95 g (51,4 mmoles) d'hydrure de lithium et d'aluminium. Après distillation (≃100 Pa, 135°C) on obtient 4,22 g d'amine.

c) *N*-(indan-2-yl)-*N*-propyl-1-benzoylpipéridine-4-méthanamine, chlorhydrate.

On chauffe à 170°C pendant 2h un mélange de 2,28 g (13 mmoles) de *N*-propylindan-2-amine et 5,0 g (14,4 mmoles) de 4-méthylphénylsulfonate de (1-benzoylpipéridin-4-yl)méthyle. On laisse le mélange revenir à température ambiante, on ajoute du dichlorométhane et un excès de soude aqueuse 1N. Après dissolution complète du milieu réactionnel, on sépare la phase organique, on évapore le solvant et on purifie le résidu par chromatographie sur colonne de gel de silice en éluant avec un mélange 97/3 de dichlorométhane/méthanol. On obtient 1,48 g d'huile visqueuse. On ajoute 2,3 ml d'acide chlorhydrique 1,7M dans l'éthanol, on agite le mélange pendant 30mn, on l'évapore à sec, et on recristallise le solide dans l'acétone. On isole finalement 0,61 g de chlorhydrate.
Point de fusion : 174-176°C.

Exemple 3

*N*-(1,2,3,4-tétrahydronaphtalén-2-yl)-*N*-méthyl-1-benzoylpipéridine-4-méthanamine, chlorhydrate.

a) 3,4-dihydro-(1*H*)naphtalén-2-oxime.

On chauffe au reflux pendant 1h un mélange de 12,34 g (84,4 mmoles) de 3,4-dihydro-(1*H*)naphtalén-2-one, 15,13 g (184,4 mmoles) d'acétate de sodium et 9,97 g (143,5 mmoles) de chlorhydrate d'hydroxylamine dans 250 ml d'éthanol absolu. On concentre le mélange, on ajoute de l'eau et de l'éther éthylique, on sépare la phase organique, on la lave à l'eau, on la sèche sur sulfate de sodium, et on évapore le solvant. On obtient 13,45 g d'huile orangée.

b) 1,2,3,4-tétrahydronaphtalén-2-amine, chlorhydrate.

A 13,45 g (83,4 mmoles) de 3,4-dihydro-(1*H*)naphtalén-2-oxime on ajoute 55 ml d'éthanol chlorhydrique

1,7M, 5 ml de chloroforme et 0,3 g d'oxyde de platine, on agite le mélange, puis on le soumet à une hydrogénation sous une pression d'environ 0,31 MPa pendant 4h à température ambiante, puis pendant 10h à 35°C. On filtre le mélange, on évapore partiellement le filtrat et on filtre le solide blanc qui précipite. Après séchage on obtient 3,22 g de chlorhydrate.

c) 1,2,3,4-tétrahydronaphtalène-2-carbamate de phénylméthyle.

On agite pendant 30mn un mélange de 11,0 g de chlorhydrate de 1,2,3,4-tétrahydronaphtalén-2-amine, 200 ml de dichlorométhane et 17,6 ml, soit 12,8 g (126 mmoles) de triéthylamine. Puis on ajoute lentement 10,3 ml (72,2 mmoles) de chloroformiate de phénylméthyle, et on poursuit l'agitation pendant 12h. On ajoute de l'eau glacée et un excès d'acide chlorhydrique 1N. On filtre le précipité, on le lave, on le sèche et on le purifie par chromatographie sur colonne de gel de silice en éluant avec un mélange 80/20 de dichlorométhane/acétate d'éthyle. On isole 12,3 g de carbamate.

d) *N*-méthyl-1,2,3,4-tétrahydronaphtalén-2-amine.

On ajoute par portions 7,43 g (26,4 mmoles) de 1,2,3,4-tétrahydronaphtalène-2-carbamate de phénylméthyle à une suspension de 4,5 g (26,4 mmoles) d'hydrure de lithium et d'aluminium dans 200 ml de tétrahydrofurane, et on chauffe le mélange au reflux pendant 3h30, on le laisse refroidir, on l'hydrolyse avec un excès de soude aqueuse 1N, on filtre le mélange, on le concentre et on le distille sous environ 80 Pa entre 140 et 240°C. On obtient 4,78 g d'huile impure qu'on utilise telle quelle dans l'étape suivante.

e) *N*-(1,2,3,4-tétrahydronaphtalén-2-yl)-*N*-méthyl-1-benzoylpipéridine-4-méthanamine, chlorhydrate.

Aux 4,78 g d'amine obtenus précédemment on ajoute 10,7 g (32 mmoles) de 4-méthylphénylsulfonate de (1-benzoylpipéridin-4-yl)méthyle et on chauffe le mélange au bain d'huile à 140°C pendant 3h. On laisse le mélange revenir à température ambiante, on ajoute du dichlorométhane et un excès de soude aqueuse 1N. Après dissolution complète du milieu réactionnel, on sépare la phase organique, on évapore le solvant et on purifie le résidu par chromatographie sur colonne de gel de silice en éluant avec un mélange 97/3 de dichlorométhane/méthanol. On obtient 4,02 g de composé encore impur auquel on ajoute 7 ml d'acide chlorhydrique 1,7M dans l'éthanol, on agite le mélange pendant 30mn, on l'évapore à sec, et on recristallise le solide dans un mélange de toluène et d'acétonitrile, puis dans l'acétone. On isole finalement 0,84 g de chlorhydrate cristallin blanc.

Point de fusion : 223-225°C.

## Exemple 4

*N*-(1,2,3,4-tétrahydronaphtalén-2-yl)-*N*-propyl-1-benzoylpipéridine-4-méthanamine, chlorhydrate.

En opérant comme dans l'exemple 3, mais en utilisant la *N*-propyl-1,2,3,4-tétrahydronaphtalén-2-amine comme amine de formule générale (III) (préparée à partir de chlorhydrate de 1,2,3,4-tétrahydronaphtalén-2-amine, selon la méthode décrite dans l'exemple 2, a et b), on obtient le chlorhydrate de *N*-(1,2,3,4-tétrahydronaphtalén-2-yl)-*N*-propyl-1-benzoylpipéridine-4-méthanamine.

Point de fusion : 200-202°C.

Le tableau qui suit illustre les structures chimiques et les propriétés physiques de quelques composés selon l'invention.

Tableau

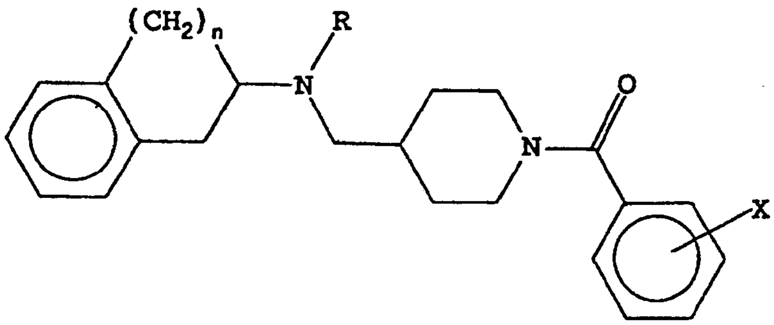

(I)

| N° | n | R | X | Sel | F(°C) |
|---|---|---|---|---|---|
| 1 | 1 | $CH_3$ | H | HCl | 212-213 |
| 2 | 1 | $\underline{n}C_3H_7$ | H | HCl | 174-176 |
| 3 | 2 | $CH_3$ | H | HCl | 223-225 |
| 4 | 2 | $\underline{n}C_3H_7$ | H | HCl | 200-202 |
| 5 | 1 | $\underline{n}C_3H_7$ | 4-F | HCl | 190-192 |
| 6 | 1 | $\underline{n}C_3H_7$ | 3-Cl | HCl | 151-154 |
| 7 | 1 | $\underline{n}C_3H_7$ | $3-CH_3$ | HCl | 147-148 |
| 8 | 1 | $\underline{n}C_3H_7$ | $3-OCH_3$ | HCl | 188-190 |
| 9 | 1 | $\underline{n}C_3H_7$ | $3-OC_2H_5$ | HCl | 200-203 |

Note : dans la colonne "R", "$\underline{n}C_3H_7$" désigne un groupe n-propyle ; dans la colonne "Sel", "HCl" désigne un chlorhydrate.

Les composés de l'invention ont été soumis a une série d'essais pharmacologiques qui ont mis en évidence leur intérêt comme substances à activités thérapeutiques.

Ainsi ils ont fait l'objet d'une étude quant à leur affinité pour les récepteurs sérotoninergiques du type $5\text{-}HT_{1A}$ présents dans l'hippocampe du rat.

Les composés déplacent la liaison d'un ligand spécifique marqué, la [$^3$H]-hydroxy-8 di-n-propylamino-2 tétraline (désignée ci-après par "[$^3$H]-8-OH-DPAT" et décrite par Gozlan et coll., Nature, (1983), <u>305</u>, 140-142) sur les récepteurs $5\text{-}HT_{1A}$. Les animaux utilisés sont des rats mâles Sprague-Dawley de 160 à 200 g. Après décapitation on en prélève le cerveau et on excise l'hippocampe. On broie le tissu dans un appareil Ultra-Turrax Polytron® pendant 30 s à la moitié de la vitesse maximale dans 10 volumes de tampon Tris 50 mM d'un pH ajusté à 7,4 avec de l'acide chlorhydrique (soit 100 mg de tissu frais par ml). On lave les tissus homogénéisés deux fois à 4°C, en les centrifugeant à chaque fois pendant 10 mn à 48000xg et en remettant le culot en suspension dans du tampon frais refroidi. Finalement on met le dernier culot en suspension dans le tampon pour arriver à une concentration de 50 mg de tissu de départ par ml de tampon à 50 mM. On laisse ensuite incuber à 37°C pendant 10 mn.

La liaison avec la [$^3$H]-8-OH-DPAT (1 nM) est déterminée par incubation de 50 µl de suspension de membranes

dans un volume final de 250 µl de tampon contenant 10 µM de pargyline et 3 µM de paroxétine. Après une incubation de 15 mn à 37°C on récupère les membranes par filtration sur filtres Whatman GF/B® qu'on lave trois fois avec des quantités aliquotes de 5 ml de tampon glacé. On extrait les filtres dans le liquide de scintillation et on en mesure la radioactivité par scintigraphie liquide. La liaison spécifique de la [$^3$H]-8-OH-DPAT est définie comme la quantité radioactive retenue sur les filtres et pouvant être inhibée par co-incubation dans la hydroxy-5 tryptamine à 10 µM. A une concentration de 1 nM de [$^3$H]-8-OH-DPAT la liaison spécifique représente 90% de la radioactivité totale recupérée sur le filtre.
Pour chaque concentration de composé étudié on détermine le pourcentage d'inhibition de la liaison avec la [$^3$H]-8-OH-DPAT, puis la concentration $CI_{50}$, concentration qui inhibe 50% de la liaison.
Pour les composés de l'invention les $CI_{50}$ se situent entre 0,001 et 0,1 µM.

L'activité centrale des composés de l'invention a été évaluée par leurs effets sur les "pointes PGO" (ponto-géniculo-occipitales) induites par la réserpine chez le chat, selon la méthode décrite par H. Depoortere dans Sleep 1976, 3rd Europ. Congr. Sleep Res., Montpellier 1976, 358-361 (Karger, Basel 1977).
On administre des doses cumulatives de composés à étudier (de 0,001 à 3 mg/kg par voie intraveineuse) à des intervalles de temps de 30 mn, 4h après injection intrapéritonéale d'une dose de 0,75 mg/kg de réserpine, à des chats curarisés, sous ventilation artificielle. On recueille les activités électroencéphalographique et phasique (pointes PGO-R) à l'aide d'électrodes corticales et profondes (genouillé latéral). Pour chaque dose de composé étudié on détermine le pourcentage de diminution du nombre de pointes PGO, puis la $DA_{50}$, dose active qui diminue de 50% ce nombre de pointes.
Pour les composés de l'invention les $DA_{50}$ se situent entre 0,001 et 1 mg/kg par la voie intraveineuse.

Les résultats des essais montrent que les composés de formule (I) possèdent, *in vitro*, une grande affinité et une sélectivité pour les récepteurs sérotoninergiques de type 5-$HT_{1A}$. *In vivo* ils montrent une activité agoniste, agoniste partielle, ou antagoniste, au niveau de ces récepteurs.

Les composés de l'invention peuvent donc être utilisés pour le traitement des maladies et affections impliquant les récepteurs sérotoninergiques de type 5-$HT_{1A}$ de façon directe ou indirecte, notamment pour le traitement des états dépressifs, des états d'anxiété, des troubles du sommeil, pour la régulation de la prise de nourriture, pour le traitement ou la prévention des vomissements et du mal des transports, et également pour le traitement de désordres vasculaires ou cardiovasculaires tels que la migraine et l'hypertension.

A cet effet ils peuvent être présentés sous toutes formes appropriées à leur administration par voie orale ou parentérale, associés à tous excipients convenables, et dosés pour permettre une posologie journalière de 1 à 1000 mg.

## Revendications

1. Composés de formule générale (I)

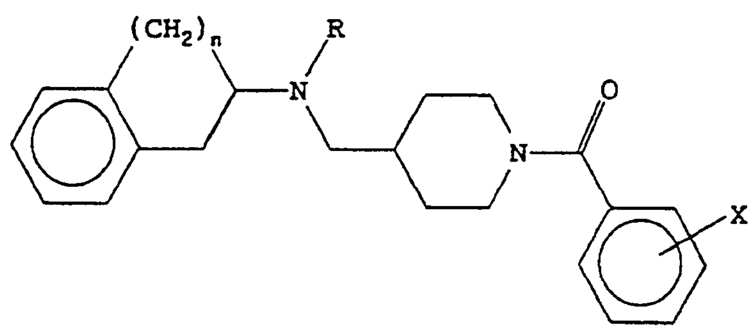

(I)

dans laquelle
n représente le nombre 1 ou 2, et
R représente un groupe alkyle linéaire ou ramifié en $C_1$-$C_3$, et
X représente un ou plusieurs substituants choisis parmi les atomes d'hydrogène et d'halogène et les groupes alkyles en $C_1$-$C_3$ et alcoxy en $C_1$-$C_3$,
à l'état de bases libres ou de sels d'addition à des acides.

2. Procédé de préparation des composés selon la revendication 1, caractérisé en ce qu'on fait réagir d'abord une amine primaire de formule générale (II)

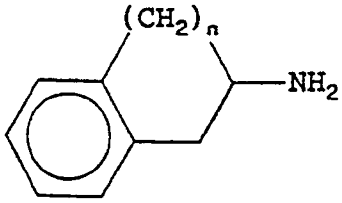

(II)

(dans laquelle n est tel que défini dans la revendication 1), soit (a) avec un chloroformiate d'alkyle ou de benzyle, soit (b) avec un chlorure d'acide en $C_2$ ou $C_3$, puis on réduit le carbamate (a) ou l'amide (b) ainsi obtenu, pour aboutir à une amine secondaire de formule générale (III)

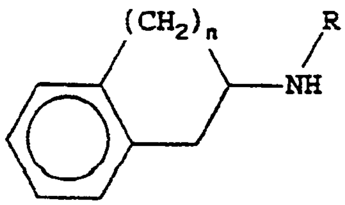

(III)

(dans laquelle R est tel que défini dans la revendication 1), et enfin on fait ensuite réagir cette amine avec un tosylate de formule générale (IV)

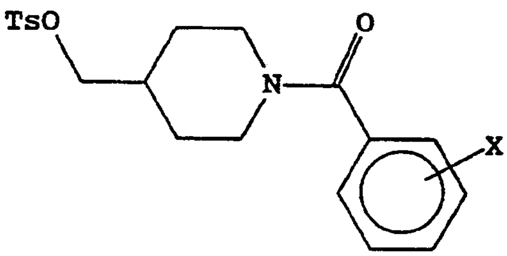

(IV)

(dans laquelle Ts représente un groupe (4-méthylphényl)sulfonyle et X est tel que défini dans la revendication 1).

3. Médicament caractérisé en ce qu'il contient un composé selon la revendication 1.

4. Composition pharmaceutique caractérisée en ce qu'elle contient un composé selon la revendication 1 associé à un excipient pharmaceutique.

**Revendication pour les états contractants : ES, GR**

1. Procédé de préparation de composés de formule générale (I)

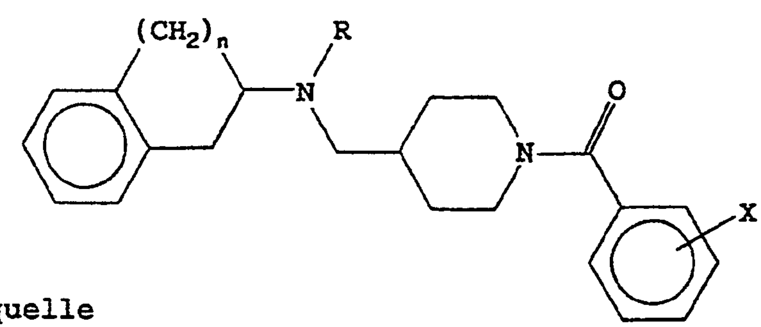

(I)

dans laquelle

n représente le nombre 1 ou 2, et
R représente un groupe alkyle linéaire ou ramifié en $C_1$-$C_3$, et

X représente un ou plusieurs substituants choisis parmi les atomes d'hydrogène et d'halogène et les groupes alkyles en $C_1$-$C_3$ et alcoxy en $C_1$-$C_3$,
procédé caractérisé en ce qu'on fait réagir d'abord une amine primaire de formule générale (II)

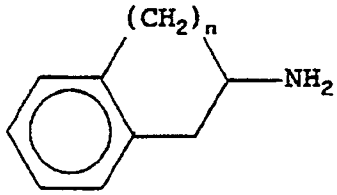

(II)

(dans laquelle n est tel que défini ci-dessus), soit (a) avec un chloroformiate d'alkyle ou de benzyle, soit (b) avec un chlorure d'acide en $C_2$ ou $C_3$, puis on réduit le carbamate (a) ou l'amide (b) ainsi obtenu, pour aboutir à une amine secondaire de formule générale (III)

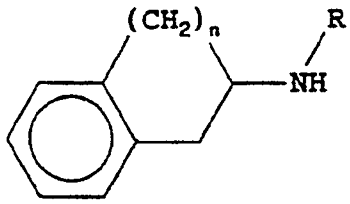

(III)

(dans laquelle R est tel que défini ci-dessus), et enfin on fait ensuite réagir cette amine avec un tosylate de formule

générale (IV)

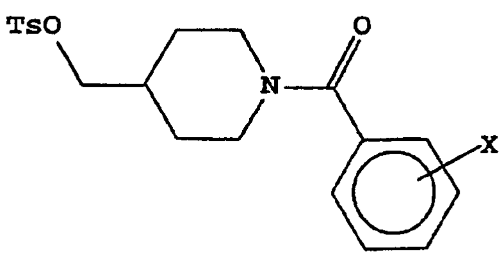

(IV)

(dans laquelle Ts représente un groupe (4-méthylphényl)sulfonyle et X est tel que défini ci-dessus).

Office européen des brevets

# RAPPORT DE RECHERCHE EUROPEENNE

Numero de la demande

EP 91 40 0585

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | CHEMICAL ABSTRACTS, vol. 51, colonne 1477, résumé no. 1477e, Columbus, Ohio, US; K.I. COLVILLE et al.: "The ganglionic-blocking activity of a series of 4-amino-ethylpiperidine dervatives", & J. AM. PHARM. ASSOC. 45, 727-9(1956)<br>* En entier *<br>--- | 1,3,4 | C 07 D 211/26<br>A 61 K 31/445 |
| A | US-A-3 634 410 (NIELSEN & FREY)<br>* En entier *<br>----- | 1,3,4 | |

| DOMAINES TECHNIQUES RECHERCHES (Int. Cl.5) |
|---|
| C 07 D 211/00 |

Le présent rapport a été établi pour toutes les revendications

| Lieu de la recherche | Date d'achèvement de la recherche | Examinateur |
|---|---|---|
| LA HAYE | 17-06-1991 | KISSLER B.E. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)